# EUROPEAN PATENT APPLICATION

(11) **EP 2 674 758 A1**
(43) Date of publication of application: **18.12.2013**
(21) Application number: 13166932.7
(22) Date of filing: 08.05.2013
(51) Int. Cl.: G01N 33/68

(54) **A computational method for mapping peptides to proteins using sequencing data**

(30) Priority: 13.06.2012 US 201213495598
(71) Applicant: Agilent Technologies, Inc., Santa Clara, CA 95051 (US)
(72) Inventor: Janis, Michael, Loveland, Colorado 80537-0599 (US); Reddy, Yugandhar, Loveland, Colorado 80537-0599 (US)
(74) Representative: Grünecker, Kinkeldey, Stockmair & Schwanhäusser

(57) **Abstract**

A method for proteomic analysis of a biological sample is disclosed, which includes obtaining peptide sequences of proteins in a target list; and identifying proteins in the biological sample by mapping the obtained peptide sequences on proteins in a proteomic database, wherein the target list is determined using information of RNA transcripts in the biological sample. The peptide sequences are determined using a mass spectrometer. The mapping is performed on a subset of proteins based on the information of RNA transcripts.

## Description

### BACKGROUND OF INVENTION

### Field of the Invention

The present invention relates to proteomics, particularly relates to workflow for protein analysis using mass spectrometer data.

### Background Art

The recent push for personalized medicine requires more efficient profiling of various biomolecules in individuals. This often involves profiling a patient's proteome. Proteome can be defined as proteins expressed by a genome. Protein expressions (hence proteome) can vary over time, for example, with changes in the individual's physiological conditions or with the presence of a pathology or a drug treatment.

Proteome analyses are typically performed with liquid chromatography (LC) coupled with tandem mass spectrometry (MS/MS). These techniques have become important tools for protein identification because they can rapidly identify complex mixtures of proteins with high sensitivities. In a typical approach, a mixture of proteins is digested (typically with trypsin) into peptides, fractionated (e.g., using LC), and analyzed by mass spectrometry (MS/MS). The resulting MS data of the tryptic fragments are then used to infer or identify the parent proteins, which may be accomplished by automated database searches.

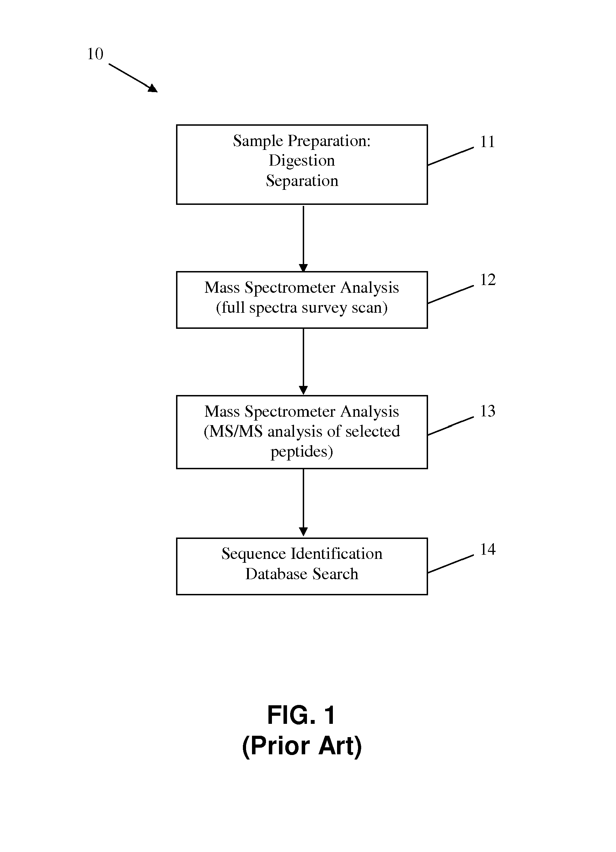

FIG. 1 shows a flowchart illustrating a general process of a proteomic analysis. As shown a method 10 starts with preparation of a sample for analysis (step 11), which may include digestion of the proteins (typically with trypsin) and separation of the fragments. The digested peptide fragments are then subjected to mass spectrometer analysis (step 12), which typically comprises a full scan (or survey scan) of all peptide fragments. Some peptide fragments (e.g., more abundant fragments) are subjected to tandem mass spectrometer (MS/MS) analysis to elucidate the peptide sequences (step 13). The peptide sequences are then used to search a database to identify the parent proteins (step 14).

This approach theoretically can lead to the identification of all proteins in the sample. This approach may be referred to as a shotgun approach. However, identification of proteins using this "shotgun" approach is extremely challenging and requires huge resources. An alternative is to analyze only proteins in a target list, in an approach referred to as targeted proteomic analysis.

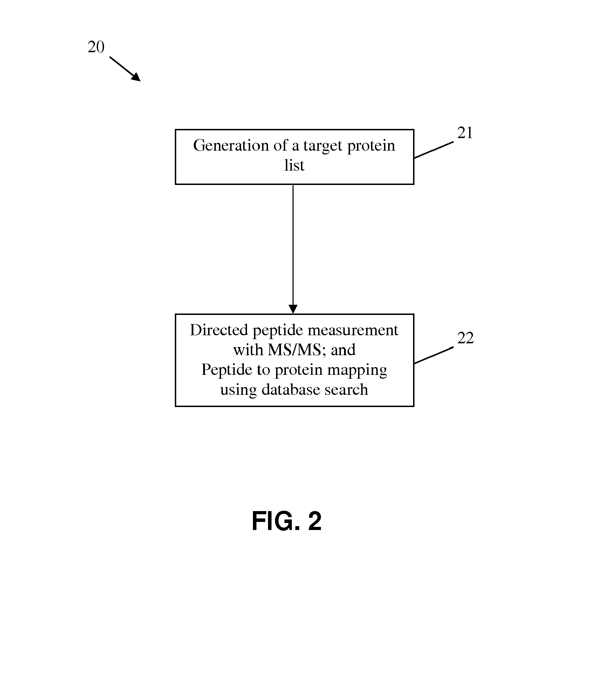

Processing of mass spectrometric data for targeted proteomic analysis typically involves two main steps: identification of target proteins, and mass spectrometer analysis of the target proteins, as illustrated in FIG. 2, which shows a method 20 comprising the step of generating a list of target proteins for analysis (step 21), followed by protein analysis with mass spectrometry (e.g., MS/MS) and peptide to protein mapping (i.e., searching a database using the peptide sequences; step 22).

Generation of a target protein list (step 21) is typically based on a survey mass spectrometer analysis to identify the proteins or peptides of interest based on relative abundance or other differential characteristics. Directed peptide measurements using MS/MS and peptide mapping (step 22) may involve the same procedures as outlined in FIG. 1, except that one would perform MS/MS measurements on the proteins in the list.

Both shotgun proteomic approach (FIG. 1) and targeted proteomic approach (FIG. 2) identify proteins by searching databases using the measured peptide sequences and infer protein abundance through assembly of measured peptide abundances. These processes typically involve large databases upon which digested peptides are mapped to likely proteins. This approach suffers from false positive mappings and the confounding presence of a large number of potential isoforms, which are difficult to disambiguate solely from the protein database. Mis-assembly of predicted proteins from peptide data therefore contains false positives. This may result in a large number of putative protein predictions, which can be challenging to analyze in a biological context. Furthermore, in targeted mass spectrometric proteomic approaches, target peptide identification is traditionally based on correlated abundance measurements, which may result in loss of target specificity. This leads to falsely targeted peptides which further complicate the peptide to protein mapping at the MS/MS phase.

### SUMMARY OF INVENTION

One aspect of the invention relates to methods for proteomic analysis in a biological sample. A method in accordance with one embodiment of the invention includes obtaining peptide sequences of proteins in a target list; and identifying proteins in the biological sample by mapping the obtained peptide sequences on proteins in a proteomic database, wherein the target list is determined using information of RNA transcripts in the biological sample and/or the target list is determined using information of RNA transcripts in the biological sample.

In accordance with some embodiments, the peptide sequences may be determined using a mass spectrometer. The target list may be determined using the information of RNA transcripts.

In accordance with some embodiments of the invention, the mapping may be performed on a subset of proteins in the proteomic database, wherein the subset of proteins is selected based on the information of RNA transcripts in the biological sample. The proteins identified may be checked against the information of the RNA transcripts to remove proteins not corresponding to any in the RNA transcripts.

In accordance with some embodiments of the invention, the proteins identified may be checked against confidence indices for the RNA transcripts. The confidence indices are obtained by a process comprising: (i) correlating each of the RNA transcripts with a protein aggregate expression level predicted from the RNA transcripts; (ii) correlating each of the RNA transcripts with aggregate proteins as derived from proteomic measurements; and (iii) deriving the confidence indices for the RNA transcripts based on comparing of correlation results from step (i) and correlation results from step (ii).

In any of the above embodiments, the information of RNA transcripts in the biological sample may be used to determine the target list of proteins. The target list may be determined based on further information of the biological system. The further information may comprise differential expression of proteins under two or more conditions. The differential expression of proteins may be determined using mass spectrometers or 2D gel electrophoresis.

The RNA transcripts in any of the above embodiments may be messenger RNA (mRNA). The mapping in the above embodiments may be performed on a subset of proteins in the proteomic database, wherein the subset of proteins is selected based on the information of mRNA transcripts in the biological sample.

Another aspect of the invention relates to methods for transcriptomic analysis of a biological sample. A method in accordance with one embodiment of the invention includes: performing proteomic analysis to obtain proteomic data comprising identities and relative abundance of proteins in the biological sample; and designing a transcriptome or genome study using the proteomic data, wherein the proteomic data are used to design sequence enrichment from a DNA library or to design a DNA microarray.

Other aspects and advantages of the invention will be apparent from the following description and the appended claims.

### BRIEF DESCRIPTION OF DRAWINGS

FIG. 1 shows schematic illustrating a process of a conventional proteomic approach.

FIG. 2 shows a workflow of a targeted proteomic approach.

FIG. 3 shows a workflow of a method in accordance with one embodiment of the invention, in which transcript sequence information is used to assist with a protein target list determination and/or to assist with protein identification using peptide sequence mapping onto proteins in a database.

FIG. 4 shows a workflow of a method in accordance with one embodiment of the invention, in which mRNA information is used to assist with a protein target list determination and/or to assist with protein identification using peptide sequence mapping onto proteins in a database.

FIG. 5 shows a flow chart illustrating a method of correlating the transcripts with proteins in accordance with one embodiment of the invention.

FIG. 6 shows a general computer that may be used with embodiments of the invention.

### DETAILED DESCRIPTION

As noted above, conventional proteomic approaches may have problems with false positive and false negative identifications. Embodiments of the invention relate to methods for novel proteomic approaches with improved accuracy. Methods of the invention use a novel joint analysis workflow that includes a targeted proteomic approach and a transcriptomic sequencing approach. In accordance with embodiments of the invention, information from trascriptomics (or "transcriptomic data") may be used to facilitate the selection of target proteins in a targeted proteomics or to improve the accuracy of protein identification using the peptide sequences in database searches. The transcriptomic data may include, for example, dominant transcript isoforms, relative abundance information, primary genomic sequence identity, single and multiple polymorphisms, insertions, deletions, and frame shifts.

In accordance with embodiments of the invention, to improve the accuracy of peptide mapping and peptide identification for targeted proteomics, methods of the invention use a joint analytical workflow that makes use of sample-specific transcript sequence information with co-measured sequence abundance measurements.

The "transcript sequence information" or "information of RNA transcripts" as used herein means the sequence information of a transcriptome in a cell. As commonly known in the art, a transcriptome may refer to all RNA molecules in a cell (including mRNA, rRNA, tRNA and other non-coding RNA products) or a subset of RNA molecules in a cell, such as mRNA molecules.

The transcript sequence information may be obtained using any sequencing and/or quantification techniques known in the art, such as DNA microarrays or the next generation sequencing (NGS) techniques (including RNA-seq). To sequence RNA, the usual method is to reverse transcribe the sample to generate cDNA molecules, which can then be sequenced using DNA sequencing techniques, whether convention sequencing techniques or the next generation sequencing (NGS) techniques.

RNA-seq refers to the use of NGS high throughput sequencing technologies to sequence cDNA to get information about RNA contents in a sample. RNA-seq provides researchers with efficient ways to measure transcriptome data (such as information on how different alleles of a gene are expressed), to detect mutations, or to identify gene fusions.

Once the RNA-seq NGS data are available, one can analyze these data using various commercially available programs (e.g., GeneSpring^{™} from Agilent Technologies) to derive information such as dominant transcript isoforms, relative abundance information, and primary genomic sequence identity. These derived data in turn can be used in any suitable proteomic programs (e.g., Agilent's Mass Qual^{™}, Mass Hunter^{™} and Mass Profiler^{™} Professional software products) for proteomic analysis, such as to design target protein lists and/or to corroborate peptide-to-protein mappings.

In accordance with embodiments of the invention, the information from transcript sequencing may ne used in two analytical approaches:
1. This information may be used to augment the differential abundance information at the peptide level, increasing the accuracy of the targeted peptides for inclusion in MS/MS analysis. This may be accomplished using any suitable proteomic programs, such as the Mass Profiler Professional^{™} and Mass Qual^{™} (available from Agilent Technologies); and/or
2. Following MS/MS analysis, the sequence information may be compared to protein prediction results (e.g., proteins predicted using Mass Hunter^{™} Qualitative software, Agilent Technologies) to prune the list of protein candidates. This would increases high-confidence protein inference results by matching the protein sequence with NGS transcript isoform measured data.

In accordance with embodiments of the invention, the transcript information may be used in either or both of the above-described steps (i.e., in the initial protein target list design and/or in the follow-up sequence confirmation). In the target list design, this software approach can ensure accurate target list generation for MS/MS analysis, while in the follow up sequence confirmation of predicted proteins, this approach may provide a strong filter against false negative peptide target list inclusion for MS/MS studies and false positive reduction in protein prediction algorithms. The use of the transcript sequence information in follow-up sequence confirmation may also be applied in shotgun proteomic approaches.

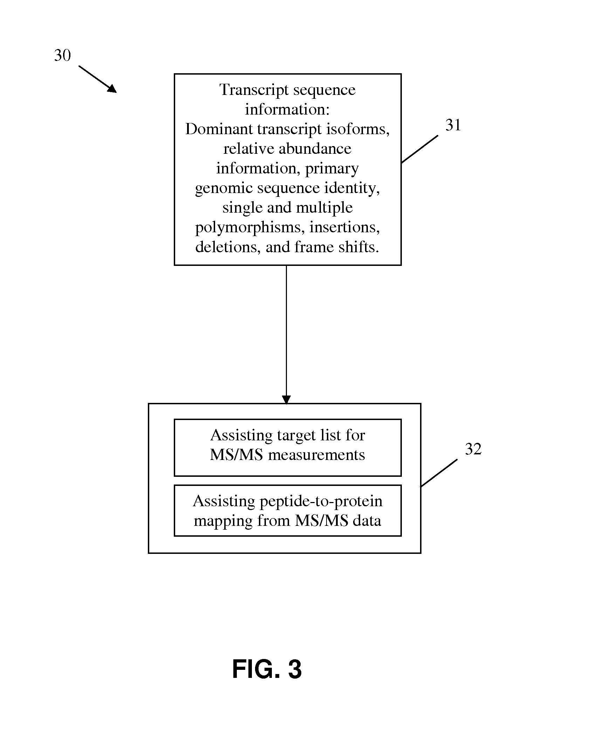

A method of the invention that uses transcriptomic information in a directed proteomic approach is illustrated in FIG. 3. As shown in FIG. 3, a method 30 involves obtaining RNA transcript sequence information (step 31). The transcript sequence information may include, for example, dominant transcript isoforms, relative abundance information, primary genomic sequence identity, single and multiple polymorphisms, insertions, deletions, and frame shifts. The transcript sequence information is then used in a targeted proteomic approach (step 32), to assist the generation of a target protein list for MS/MS measurements and/or to assist the peptide to protein mapping (i.e., database search) from the MS/MS data (i.e., using peptide sequence data).

The protein database that can be used for the search may be any of those used for conventional proteomics, such as human protein reference database, Nation Center for Biotechnology Information (NCBI), protein data bank (PDB), protein information resource (PIR), proteomics identification database (PRIDE), Swiss-Prot, or UniProt. These databases are well known to one skilled in the art.

In accordance with some embodiments of the invention, the transcriptomic information may include only a subset of RNA molecules, such as mRNA, in a biological sample. A transcriptome that includes only mRNA molecules may have more direct relationship with the proteome in the biological sample.

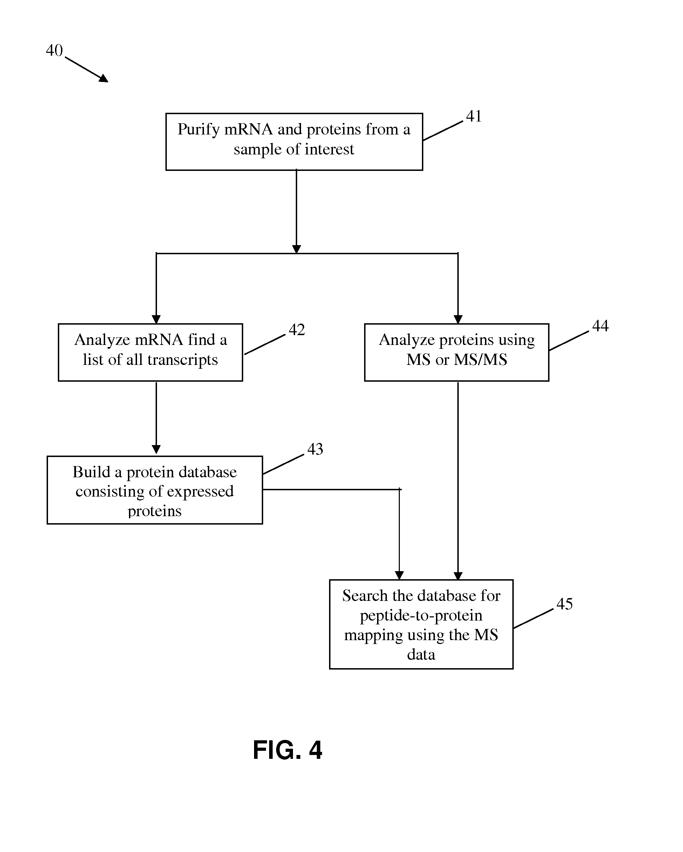

FIG. 4 shows a method in accordance with embodiments of the invention that uses mRNA transcriptomic information in conjunction with proteomic information. As shown in FIG. 4, a method 40 may include purifying mRNA and proteins from a sample of interest (step 41). The purification may be performed with techniques known in the art, including using commercially available kits..

Then, the mRNA molecules are analyzed to identify a list of all transcripts that are present in the sample regardless of the level of expression (step 42). Analysis of mRNA may be performed with any techniques known in the art, such as DNA microarrays. Based on the list of all transcripts, a protein database may be built consisting of all expressed proteins (step 43).

The proteins purified from the sample may be analyzed using mass spectrometer (e.g., MS/MS) to produce the proteomic information (step 44). The mass spectrometer analysis may be performed with all proteins in the sample or with proteins in a target list that is established with aid of the mRNA information. Finally, the proteomic information (e.g., the peptide sequences) is then searched against the protein database, which has been built using the mRNA transcript information (see step 43), to identify the proteins (step 45).

As noted above, the transcript information may be used to check or confirm the proteins identified from peptide-to-protein mapping (searching a database). The transcript information may be used in a qualitative sense, whether a particular protein is present or absent in the biological sample, to help reduce the false positives. Alternatively, the transcript information may be used in a more quantitative sense to indicate the relative confidence of the protein identifications.

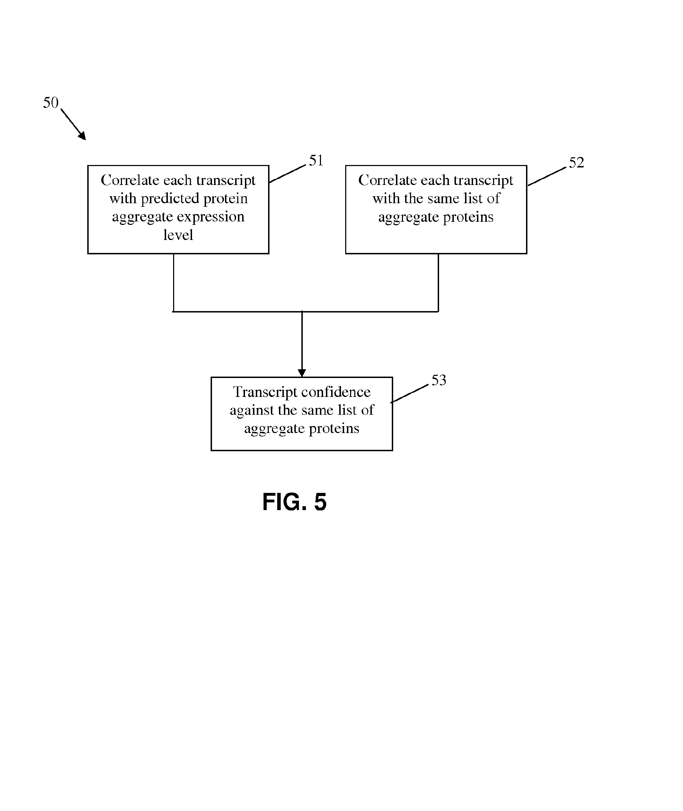

For example, to better understand the relationship of the transcriptome to the proteome, an analytical approach for transcription-to-translation correlation may be implemented. Then, the transcription-to-translation correlation may be used to augment the list of predicted protein data. In this approach, the measured data from the transcriptomic technology (such as RNA-Seq) and the peptide data from MS/MS experiments, for example, may be correlated in a process 50 shown in FIG. 5.

First, each transcript is correlated with the predicted protein aggregate expression level as derived from the transcriptomic analysis (step 51), and every transcript is also correlated with the list of aggregate proteins as derived from the proteomic analysis (step 52). Then, by comparing these correlations, a transcript confidence may be reported for each protein in the list of the aggregate proteins (step 53). In accordance with embodiments of the invention, these transcript confidence indices may be used to confirm or evaluate the reliability (confidence) of the peptide-to-protein mapping in the database search.

In addition, the mapping of these correlated values, rather than the raw or normalized values, may be applied at the biological network or pathway level to elucidate the mechanisms of cellular responses for both the transcriptome and the proteome.

Although the transcriptomic and proteomic data needed for the approaches described above may be obtained using RNA-seq or NGS for the transcriptomic data and MS technologies for the proteomic data, it should be noted that the transcriptomic and proteomic data needed for use with embodiments of the invention may be obtained using any suitable techniques. In other words, embodiments of the invention are based on a generalized methodology that relies on sequence level data from the transcriptome and peptide level data from the proteome. This generalized methodology can be used with a variety of measurement technologies. For example, for proteomic data, one may use other peptide sequencing technologies (e.g., Edman degradation) or protein identification technologies (e.g., antibodies, or ELISA (enzyme-linked immunosorbent assay)), and for transcriptomic data, one may use microarray technologies.

Embodiments of the invention described above are based on *a priori* transcriptomic data to assist the analysis of proteomic data. These methods are based on relationship between the transcriptomic data and the proteomic data. In a similar manner, this relationship may be used in the reversed direction. Thus, some embodiments of the invention relate to methods of using *a priori* proteomic data (e.g., from MS analysis) to assist the analysis of transcriptomic data, for example, to increase the quality of the measured data from microarray and/or next-generation sequencing experiments. In this approach, the proteomics data may be used as *a priori* knowledge for the design of matched experiments using targeted genomic enrichment or custom microarrays. Such a computation design of experiment approach will find value in biomarker discovery, cancer research, and Toxicogenomics studies.

For example, in accordance with embodiments of the invention, proteomic data may be used to help design enriched genomic library or design custom microarray. An example of a genomic library enrichment systems is the SureSelect^{™} system available from Agilent Technologies, Inc. (Santa Clara, CA). The SureSelect^{™} system uses a biotin-avidin based selection technique (i.e., biotinylated bait sequences and steptavidin coated magnetic beads) to enrich the sequences of interest. This system can significantly improve the cost and process efficiency of a sequencing workflow. In accordance with embodiments of the invention, the proteomic data may be used to help design the enrichment sequences (the bait sequences) to enrich a library.

Methods of the invention may be incorporated into existing software that is available for genomic, proteomic, or multi-omic data analysis. In particular, the correlation between the transcriptomic data and the proteomic data can be conveniently performed using a multi-omic (genomics, proteomics, transcriptomics, etc.) software. One example of a multi-omic software that can be used with methods of the invention is the GeneSpring^{™} analytical platform available from Agilent Technologies. GeneSpring^{™} analytical platform is a multi-omics software solution for the practical integration of cellular measurement data from different knowledge domains. Sucha multi-omic analytical approach may be used with methods of the invention to increase the quality of the measured data from mass spectrometric proteomic experiments using next-generation sequencing data (transcriptomic data), or to increase the quality of the measured data from microarray and/or next-generation sequencing experiments using the proteomic data.

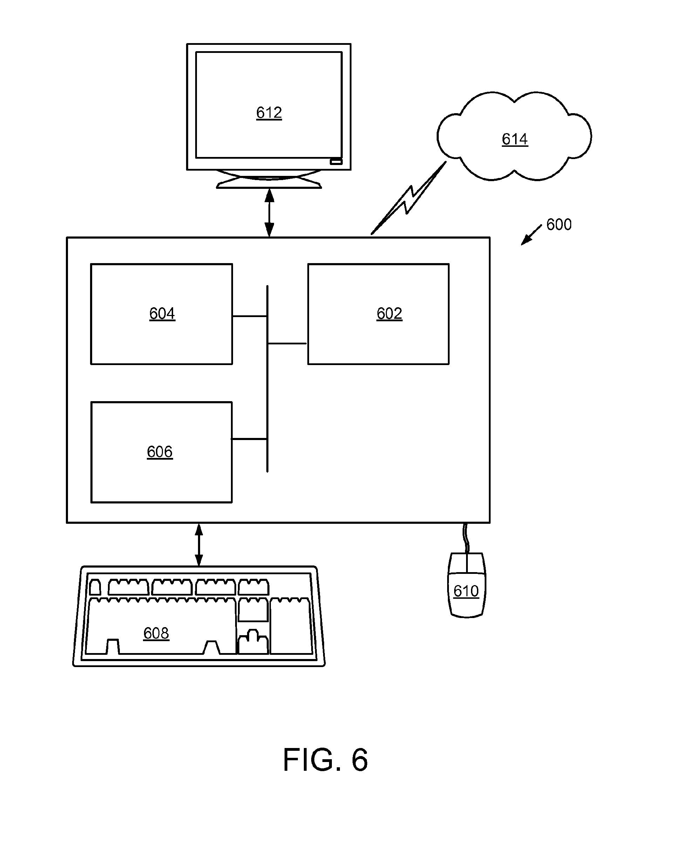

Embodiments of the invention may be implemented on virtually any type of computer regardless of the platform being used. For example, as shown in FIG. 6, a computer system (600) includes one or more processor(s) (602), associated memory (604) (e.g., random access memory (RAM), cache memory, flash memory, etc.), a storage device (606) (e.g., a hard disk, an optical drive such as a compact disk drive or digital video disk (DVD) drive, a flash memory stick, etc.), and numerous other elements and functionalities typical of today's computers (not shown). The computer (600) may also include input means, such as a keyboard (608), a mouse (610), or a microphone (not shown). Further, the computer (600) may include output means, such as a monitor (612) (e.g., a liquid crystal display (LCD), a plasma display, or cathode ray tube (CRT) monitor). The computer system (600) may be connected to a network (614) (e.g., a local area network (LAN), a wide area network (WAN) such as the Internet, or any other similar type of network) via a network interface connection (not shown). Those skilled in the art will appreciate that many different types of computer systems exist, and the aforementioned input and output means may take other forms. Generally speaking, the computer system (600) includes at least the minimal processing, input, and/or output means necessary to practice embodiments of the invention.

Further, those skilled in the art will appreciate that one or more elements of the aforementioned computer system (600) may be located at a remote location and connected to the other elements over a network. Further, embodiments of the invention may be implemented on a distributed system having a plurality of nodes, where each portion of the invention (e.g., display, formation data, analysis device, etc.) may be located on a different node within the distributed system. In one embodiment of the invention, the node corresponds to a computer system. Alternatively, the node may correspond to a processor with associated physical memory. The node may alternatively correspond to a processor with shared memory and/or resources. Further, software instructions to perform embodiments of the invention may be stored on a computer readable medium such as a compact disc (CD), a diskette, a tape, a file, or any other computer readable storage device.

Advantages of embodiments of the invention may include one or more of the following. Embodiments of the invention use a multi-omic approach to help increase the accuracy of proteomic analysis. Methods of the invention use transcriptomic data to assist the generation of target protein list and/or to improve protein identification using peptide-to-protein mapping. Methods of the invention use a generalized methodology that is based on transcriptome level data and proteome level data, regardless what techniques are used to obtain these data. Therefore, methods of the invention may be used with variety of technologies.

While the invention has been described with respect to a limited number of embodiments, those skilled in the art, having benefit of this disclosure, will appreciate that other embodiments can be devised which do not depart from the scope of the invention as disclosed herein. Accordingly, the scope of the invention should be limited only by the attached claims.

## Claims

1. A method for proteomic analysis of a biological sample, comprising:
obtaining peptide sequences of proteins in a target list; and
identifying proteins in the biological sample by mapping the obtained peptide sequences on proteins in a proteomic database,
wherein the target list is determined using information of RNA transcripts in the biological sample and/or the target list is determined using information of RNA transcripts in the biological sample.

2. The method of claim 1, wherein the peptide sequences are determined using a mass spectrometer.

3. The method of claim 1 or 2, wherein the mapping is performed on a subset of proteins in the proteomic database, wherein the subset of proteins is selected based on the information of RNA transcripts in the biological sample.

4. The method of any one of claims 1 to 3, wherein the proteins identified are checked against the information of the RNA transcripts to remove proteins not corresponding to any in the RNA transcripts.

5. The method of any one of claims 1 to 4, wherein the proteins identified are checked against confidence indices for the RNA transcripts.

6. The method of claim 5, wherein the confidence indices are obtained by a process comprising:
(i) correlating each of the RNA transcripts with a protein aggregate expression level predicted from the RNA transcripts;
(ii) correlating each of the RNA transcripts with aggregate proteins as derived from proteomic measurements; and
(iii) deriving the confidence indices for the RNA transcripts based on comparing of correlation results from step (i) and correlation results from step (ii).

7. The method of any one of claims 1 to 6, wherein the information of RNA transcripts in the biological sample is used to determine the target list, and the target list is also determined based on information of a biological system.

8. The method of claim 7, wherein the information of the biological system comprises information of differential expression of proteins under two conditions.

9. The method of claim 8, wherein the differential expression of proteins are identified by 2-dimensional gel electrophoresis or by mass spectrometer analysis.

10. The method of any one of claims 1 to 9, wherein the information of RNA transcripts comprises information of messenger RNA (mRNA) transcripts.

11. The method of claim 10, wherein the mapping is performed on a subset of proteins in the proteomic database, wherein the subset of proteins is selected based on the information of mRNA transcripts in the biological sample.

12. A method for transcriptomic analysis of a biological sample, comprising:
performing proteomic analysis to obtain proteomic data comprising identities and relative abundance of proteins in the biological sample; and
designing a transcriptome or genome study using the proteomic data, wherein the proteomic data are used to design sequence enrichment from a DNA library or to design a DNA microarray.
